# EUROPÄISCHE PATENTSCHRIFT

(11) **EP 2 092 954 B1**
(45) Veröffentlichungstag und Bekanntmachung des Hinweises auf die Patenterteilung: **29.07.2015**
(21) Anmeldenummer: 09150979.4
(22) Anmeldetag: 21.01.2009
(51) Int. Cl.: A61N 1/05

(54) **Verfahren zur Herstellung eines Isolationsschlauchs und Verfahren zur Herstellung einer Elektrode**
Method for producing an isolation tube and method for producing an electrode
Procédé de fabrication d'un flexible isolant et procédé de fabrication d'une électrode

(30) Priorität: 20.02.2008 DE 102008010188
(43) Veröffentlichungstag der Anmeldung: 26.08.2009
(73) Patentinhaber: Biotronik CRM Patent AG, 6341 Baar (CH)
(72) Erfinder: Abdu, Akram, 14059, Berlin (DE); Zedler, Stephan, 12309, Berlin (DE); Schurr, Marc, 13359, Berlin (DE)
(74) Vertreter: Lindner-Vogt, Karin L.

(56) Entgegenhaltungen:
- EP-A2- 1 872 825
- US-A- 4 312 693
- US-A- 5 358 516
- US-A1- 2001 032 006
- US-B2- 7 221 982

## Beschreibung

Die vorliegende Erfindung betrifft ein Verfahren zur Herstellung eines Isolationsschlauchs für eine Elektrode für medizinische Anwendungen, vorzugsweise zur Implantation als Bestandteil eines funktionalen Elektrostimulationsgeräts, wobei der Isolationsschlauch eine erste, im Wesentlichen hohlzylinderförmige Schicht und mindestens eine zweite Schicht aufweist, die auf der Mantelfläche der ersten Schicht angeordnet ist und diese mindestens teilweise bedeckt. Die vorliegende Erfindung betrifft ferner ein Verfahren zur Herstellung einer vorstehend genannten Elektrode.

Eine Elektrode für medizinische Anwendungen wird vorzugsweise als Bestandteil eines funktionalen Elektrostimulationsgeräts (FES) zur Elektrobehandlung von Nerven- oder Muskelzellen im diagnostischen oder therapeutischen Bereich eingesetzt. Implantationssysteme für die Elektrostimulation umfassen beispielsweise Herzschrittmacher mit einem Impulsgenerator zur künstlichen Anregung von Herzaktionen oder Defibrillatoren. Diese Elektrostimulationsgeräte umfassen üblicherweise ein körperverträgliches Gehäuse mit einer dazugehörigen elektronischen Schaltung und einer Energieversorgung, z. B. einer Batterie. Das Gehäuse besitzt mindestens eine Anschlussstelle, an der die Elektrode oder die Elektroden angeschlossen werden können. Die Elektrode oder die Elektroden dienen der Übertragung der elektrischen Energie vom Gehäuse zu dem behandelten Gewebe und umgekehrt.

Hierbei bezeichnet der Begriff "Elektrode" in der Medizintechnik nicht nur ein Element, mit dem nach physikalischer Definition elektrische Energie übertragen wird, sondern umfasst eine Leitung mit einem elektrischen Leiter zusammen mit seiner umhüllenden Isolation, die häufig als Isolationsschlauch ausgebildet ist, sowie alle weiteren funktionellen Elemente, die mit der Leitung fest verbunden sind. Insbesondere umfasst die Elektrode auch die sogenannte Elektrodenspitze, mittels der die elektrische Energie in das zu behandelnde Gewebe eingeleitet wird. Häufig ist eine Elektrodenspitze auch mit Ankerelementen oder Haltestrukturen versehen, mit denen die Konstanz der räumlichen Lage der Übergangsstelle der elektrischen Energie in dem zu behandelnden Gewebe sichergestellt wird. Die Elektrodenspitze, die eine Übergangsstelle der elektrischen Energie in das Gewebe bildet, kann als Ableit-, Stimulations- oder Messelektrode ausgelegt sein.

Aus der Druckschrift US 7,221,982 B2 ist eine implantierbare Herzelektrode bekannt, welche einen Isolationsschlauch bestehend aus zwei hohlzylinderförmigen Schichten aufweist. Hierbei definiert die erste Schicht den isolierenden Körper der Elektrode. Die zweite Schicht, die als Schutzschicht dient, ist entlang der äußeren Oberfläche der ersten Materialschicht, und zwar lediglich entlang eines Teils dieser Oberfläche, aufgebracht. Die Oberfläche der ersten Schicht wird aktiviert, um die Haftfähigkeit zu erhöhen und die Aufbringung der Schutzschicht zu erlauben. Hierfür offenbart die Druckschrift verschiedene Verfahren. Beispielsweise kann eine Plasma-Oberflächenmodifikation durch ein plasmaassistiertes Verfahren oder eine verbesserte chemische Gasphasenabscheidungsbehandlung (chemical vapor deposition treatment) verwendet werden. Die Plasmabehandlung umfasst die Reinigung der Oberfläche mittels Gasen. Die Plasmabehandlung beinhaltet außerdem ein Plasmaabscheidungsprozess mit einem Gas, um eine Ankerschicht (tie layer) auszubilden, sowie die Funktionalisierung der Oberfläche und die Aktivierung dieser, um die Haftfähigkeit zu verbessern. Das herkömmliche Verfahren zur Herstellung einer Elektrode mit einem Isolationsschlauch ist aufwändig und kostenintensiv.

Die Aufgabe besteht demnach darin, ein Verfahren anzugeben, mit dem auf einer ersten Schicht eines Isolationsschlauchs (Unterschlauch) schnell und kostengünstig eine weitere Schicht zum Abriebschutz aufgebracht werden kann. Hierbei soll die Haftung auf dem Unterschlauch möglichst konstant und langzeitstabil ausfallen. Zudem ist es notwendig, dass das Verfahren möglichst schonend ist, um eine lange und sichere Funktion der Elektrode im behandelten Körper zu gewährleisten.

Die oben angegebene Aufgabe wird durch ein Verfahren zur Herstellung eines Isolationsschlauchs gelöst, bei dem die Mantelfläche der ersten Schicht vor dem Auftragen der mindestens einen zweiten Schicht in einem Reinigungsschritt mittels eines ersten, flüssigen Reinigungsmittels bei einer Temperatur unterhalb von 30 C, vorzugsweise unterhalb von 20 C gereinigt wird. Ein derartiges Verfahren lässt sich schnell durchführen und ist zudem kostengünstig. Durch das angegebene Verfahren werden die Eigenschaften des Schlauchs homogenisiert, die Oberfläche gereinigt und kurzkettige, nicht vernetzte Reste auf der Oberfläche des Schlauchs entfernt. Hierdurch wird erreicht, dass eine anschließend als Abriebschutz aufgebrachte, zweite Schicht besser auf dem Untergrund (d. h. der Mantelfläche der ersten Schicht) haftet. Hierbei ist die Haftung der zweiten Schicht auf dem Unterschlauch konstant und langzeitstabil. Das erfindungsgemäße Verfahren, das einen Reinigungsschritt beinhaltet, der im Folgenden auch als Kalthärten (auch Kaltegalisierung) bezeichnet wird, ist zudem schonend und gewährleistet somit, dass eine Elektrode mit einem nach dem erfindungsgemäßen Verfahren hergestellten Isolationsschlauch lange und sicher im Körper des Behandelten funktioniert.

In einem bevorzugten Ausführungsbeispiel ist das erste Reinigungsmittel flüssiges CO₂. Dieses Reinigungsmittel ist besonders kostengünstig und jederzeit verfügbar.

Besonders vorteilhaft ist, wenn nach dem Reinigungsschritt und vor dem Auftragen der mindestens einen zweiten Schicht ein Haftvermittler, der vorzugsweise mindestens eine Verbindung aus der Gruppe der Silane, Siloxane und Carbinol enthält, auf mindestens einen Teil der Mantelfläche der ersten Schicht aufgetragen wird. Ein derartiger Haftvermittler (Primer) dient dazu, die Haftung der abriebfesten zweiten Schicht auf dem Untergrund weiter zu verbessern.

In anderen Fällen kann durch das erfindungsgemäße Verfahren mit dem Kalthärten-Reinigungsschritt die Verwendung eines Haftvermittlers entfallen. Dies ist von Vorteil, da Haftvermittler biologisch oft nicht ganz unbedenklich sind. Falls die erste Schicht aus Silikon besteht, könnte ein Haftvermittler deren Eigenschaften (z. B. Elastizität und Reißfestigkeit) beeinträchtigen. Zudem lässt sich die Beschaffenheit des aufgetragenen Haftvermittlers, wenn dieser getrocknet ist, nur schwer kontrollieren. Folglich können Mängel, falls diese beim Auftragen entstanden sind, leicht übersehen werden. Durch Mängel beim Auftragen des Haftvermittlers würde die Haftung der auf den Haftvermittler aufgebrachten zweiten Schicht beeinträchtigt werden. Da ggf. durch das erfindungsgemäße Verfahren mit dem Kalthärten-Reinigungsschritt auf den Haftvermittler verzichtet werden kann, erhöht sich somit die Sicherheit des Patienten und es können weitere Kosten eingespart werden.

Es ist außerdem bevorzugt, dass nach dem Reinigungsschritt und vor dem Auftragen der mindestens einen zweiten Schicht eine Plasmavorbehandlung der Mantelfläche der ersten Schicht durchgeführt wird. Die Plasmavorbehandlung kann mit oder ohne Einsatz eines Prozessgases erfolgen und anstatt der Verwendung eines Haftvermittlers oder zusätzlich zu einem solchen erfolgen. Falls die Plasmavorbehandlung zusätzlich zur Aufbringung eines Haftvermittlers erfolgt, wird die Plasmavorbehandlung vor dem Auftragen des Haftvermittlers durchgeführt. Die Plasmavorbehandlung dient ebenso wie der Haftvermittler dazu, die Haftung der zweiten Schicht auf der ersten Schicht zu verbessern. Es können beispielsweise ein Niederdruckplasma oder verschiedene Arten von Atmosphärenplasmen zum Einsatz kommen. Beim Atmosphärenplasma wird die Verwendung eines potentialfreien offenen Atmosphärenplasmas bevorzugt. Aber auch die Verwendung einer Koronaentladung zur Erzeugung des Plasmas ist von Nutzen. Als Prozessgase sind zum Beispiel Luft und/oder ihre Bestandteile, Wassergas, Silan- und Siloxan-Verbindungen denkbar.

Die nach dem erfindungsgemäßen Verfahren gereinigte Mantelfläche der ersten Schicht wird vorzugsweise dadurch mit der mindestens einen zweiten Schicht versehen, dass die mindestens eine zweite Schicht mittels Tauchen, Aufsprühen oder Co-Extrusion auf die gereinigte erste Schicht aufgetragen wird. Diese Verfahren sind kostengünstig und eignen sich für die in Frage kommenden Materialien der mindestens einen zweiten Schicht gut.

In einem bevorzugten Ausführungsbeispiel enthält die erste Schicht Silikon. Der Vorteil einer Schicht mit Silikon besteht darin, dass Silikon ein variables, in einigen Parametern, wie zum Beispiel der Härte, sehr gut anpassbares Material ist. Dadurch lassen sich die physikalischen Eigenschaften der fertigen Elektrode gezielt an die Erfordernisse im Körper anpassen. Ein weiterer Vorteil von Silikon ist, dass es in Körper eine sehr gute Langzeitstabilität und biologische Verträglichkeit aufweist und somit die sichere elektrische Isolation der Elektrode über Jahrzehnte hinweg gewährleisten kann.

Von Vorteil ist außerdem, wenn die mindestens eine zweite Schicht mindestens ein Polymer aus der Gruppe bestehend aus Polyurethan und Silikon-Polyurethan-Copolymer enthält. Eine derartige Schicht ist besonders abriebfest und eignet sich somit hervorragend als Abriebschutz.

Die obige Aufgabe wird ferner durch ein Verfahren zur Herstellung einer Elektrode gelöst, bei dem der Isolationsschlauch nach einem oben angegebenen Verfahren hergestellt wird und anschließend der Isolationsschlauch so auf ein Leiterelement aufgebracht wird, dass es diesen außen umgibt und elektrisch isoliert. Der Isolationsschlauch wird an den Enden, an Übergangsstellen, in Teilbereichen oder über die gesamte Länge mit dem Leiterelement oder daran angebrachten Elementen verklebt oder auf andere Weise form- und/oder kraftschlüssig verbunden. Eine derartige Elektrode hat den Vorteil, dass sie einfach und kostengünstig herstellbar ist und die Funktionssicherheit der Elektrode verbessert wird.

Wie oben beschrieben kommt es durch die Beschichtung mit der zweiten Schicht zu einer Verbesserung der Gleiteigenschaften und der Handhabung während der Implantation und zu einer Erhöhung der Abriebbeständigkeit und damit auch der Langzeitstabilität und der Einsatzdauer der Elektrode im Patienten. Das liegt vor allem darin begründet, dass durch das Kalthärten in Verbindung mit der zweiten Schicht und ggf. dem Primer ein Isolationsschlauch erzeugt wird, dessen Eigenschaften in einem sehr engen Rahmen gehalten werden können. Durch das Reinigen werden kurzkettige, nicht vernetzte Reste und Schmutz vom Schlauchherstellungsprozess zum größten Teil aus dem Schlauch ausgewaschen. Dadurch haften Primer und/oder die zweite Schicht besser auf dem Untergrund. Parallel dazu egalisiert der Reinigungsprozess die Eigenschaften (Biegesteifigkeit, Zugfestigkeit, Härte, Torsionssteifigkeit, Oberflächenbeschaffenheit....) des Grundschlauchs. Ein Tempern bei hohen Temperaturen für eine längere Zeit (150-220°C für 2-5 h) wird damit überflüssig. Das Grundmaterial wird somit einer geringeren Belastung ausgesetzt. Der so egalisierte Grundschlauch hat eine optimierte Oberfläche, auf die die zweite Schicht aufgetragen werden kann. Hierdurch kann die zweite Schicht dünner gestaltet werden als bei einem herkömmlichen Schlauch, der nicht mittels Kalthärten vorbehandelt ist. Die Elektrode kann damit dünner ausfallen (erhöhte Flexibilität, mehr Platz zur Implantation weiterer Elektroden im venösen System). Es kommt durch die gleichmäßigen Eigenschaften auch zu einer weiteren Verbesserung der Langzeitstabilität und zu einer verbesserten Handhabung während der Implantation. (Die Elektrode fühlt sich immer gleich an, nur so kann gewährleistet werden, dass sich der Arzt an eine solche Zuleitung "gewöhnt" und damit schneller und effizienter implantieren kann). Konstruktiv können mehr Freiheiten in der Gestaltung der Elektroden erlangt werden, wodurch sich die Elektrode in einem stärkeren Maß als bisher an die Erfordernisse im Körper anpassen lässt. Dadurch wird eine erhöhte Patientensicherheit erzielt.

Weitere Ziele, Merkmale, Vorteile und Anwendungsmöglichkeiten der Erfindung ergeben sich aus der nachfolgenden Beschreibung der erfindungsgemäßen Verfahren anhand der Figur. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigt:

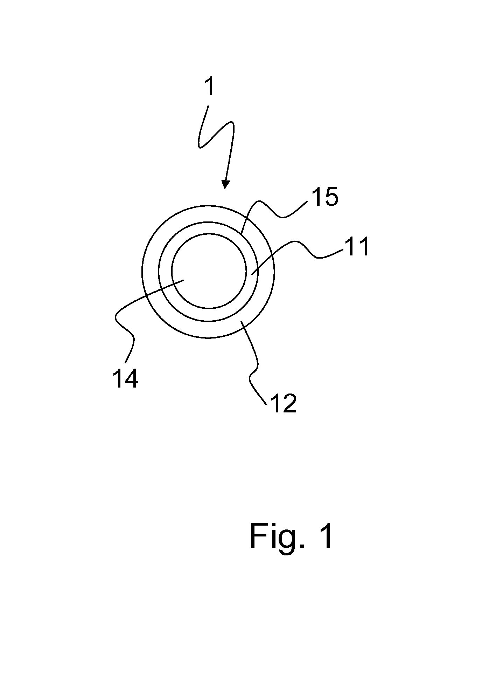

- Figur 1: ein Querschnitt eines Isolationsschlauchs für eine Elektrode für medizinische Anwendungen, hergestellt durch das erfindungsgemäße Verfahren.

Der in Figur 1 dargestellte Isolationsschlauch 1 weist eine erste Schicht (Unterschlauch) 11 auf, die im Wesentlichen hohlzylindrisch ausgebildet ist. Auf der ersten Schicht 11 ist eine zweite Schicht 12 angeordnet, wobei diese konzentrisch zur ersten Schicht verläuft. Die erste elektrisch isolierende Schicht 11 besteht vorzugsweise aus Silikon, während die zweite Schicht 12 vorzugsweise aus Polyurethan oder einem Silikon-Polyurethan-Copolymer besteht und als Abriebschutz dient. In der mittigen durchgehenden Öffnung 14 der ersten Schicht 11 werden nach der Herstellung des Isolationsschlauchs das Leiterelement oder die Leiterelemente (nicht dargestellt) der Elektrode angeordnet, die durch den Isolationsschlauch 1 elektrisch isoliert und geschützt werden. Das Leiterelement oder die Leiterelemente dienen der Übertragung der elektrischen Energie vom Gehäuse des funktionalen Elektrostimulationsgeräts (z. B. Herzschrittmacher) zu der zu behandelnden Stelle.

Zur Herstellung eines derartigen Isolationsschlauchs 1 wird zunächst die Mantelfläche 15 eines Schlauchs, der lediglich aus der ersten Schicht 11 besteht, in flüssigem CO₂ gereinigt (Kalthärten), wobei dem CO₂ ggf. ein weiteres Reinigungsmittel beigegeben sein kann. Anschließend wird mittels Tauchen, Aufsprühen oder Co-Extrusion die zweite Schicht 12 auf die Mantelfläche 15 der ersten Schicht 11 aufgebracht. Dazu kann eine Lösung aus dem Material der zweiten Schicht verwendet werden oder die Grundsubstanzen aufgesprüht werden, so dass diese direkt auf der Mantelfläche 15 des Unterschlauchs 11 polymerisieren. Ggf. kann vor dem Aufbringen der zweiten Schicht 12 und nach dem Kalthärten-Reinigungsschritt ein Haftvermittler auf die Mantelfläche 15 aufgetragen werden und/oder eine Plasmavorbehandlung der Mantelfläche 15 mit oder ohne Einsatz eines Prozessgases durchgeführt werden.

Durch das erfindungsgemäße Verfahren mit dem Kalthärten-Reinigungsschritt wird der Unterschlauch 11 gereinigt und in einen Zustand versetzt, in dem seine Eigenschaften nahezu konstant sind, so dass die Beschichtung 12 besser und definierter haftet. Dadurch erhöht sich die mechanische und biologische Stabilität der gesamten Elektrode mit dem Isolationsschlauch 1 im Körper. Hierdurch lassen sich Silikonzuleitungskörper in größeren Stückzahlen kostengünstig herstellen. Es können außerdem Schichten für den Abriebschutz realisiert werden, die dünner sind als bei herkömmlichen Elektroden.

### Bezugszeichenliste:

- 1: Isolationsschlauch
- 11: erste Schicht des Isolationsschlauchs 1
- 12: zweite Schicht des Isolationsschlauchs 1
- 14: mittige durchgehende Öffnung in der ersten Schicht 11
- 15: Mantelfläche der ersten Schicht 11

## Patentansprüche

1. Verfahren zur Herstellung eines Isolationsschlauchs (1) für eine Elektrode für medizinische Anwendungen, vorzugsweise zur Implantation als Bestandteil eines funktionalen Elektrostimulationsgeräts, wobei der Isolationsschlauch (1) eine erste, im Wesentlichen hohlzylinderförmige Schicht (11) und mindestens eine zweite Schicht (12) aufweist, die auf der Mantelfläche (15) der ersten Schicht (11) angeordnet ist und diese mindestens teilweise bedeckt, **dadurch gekennzeichnet, dass** die Mantelfläche (15) der ersten Schicht (11) vor dem Auftragen der mindestens einen zweiten Schicht (12) in einem Reinigungsschritt mittels eines ersten, flüssigen Reinigungsmittels bei einer Temperatur unterhalb von 30°C, vorzugsweise unterhalb von 20°C gereinigt wird.

2. Verfahren nach Anspruch 1, **dadurch gekennzeichnet, dass** das erste Reinigungsmittel flüssiges CO₂ ist.

3. Verfahren nach einem der vorhergehenden Ansprüche, **dadurch gekennzeichnet, dass** nach dem Reinigungsschritt und vor dem Auftragen der mindestens einen zweiten Schicht (12) ein Haftvermittler, der vorzugsweise mindestens eine Verbindung aus der Gruppe der Silane, Siloxane und Carbinol enthält, auf mindestens einen Teil der Mantelfläche (15) der ersten Schicht (11) aufgetragen wird.

4. Verfahren nach einem der vorhergehenden Ansprüche, **dadurch gekennzeichnet, dass** nach dem Reinigungsschritt und vor dem Auftragen der mindestens einen zweiten Schicht (12) eine Plasmavorbehandlung der Mantelfläche (15) der ersten Schicht (12) durchgeführt wird.

5. Verfahren nach einem der vorhergehenden Ansprüche, **dadurch gekennzeichnet, dass** die mindestens eine zweite Schicht (12) mittels Tauchen, Aufsprühen oder Co-Extrusion auf die gereinigte erste Schicht aufgetragen wird.

6. Verfahren nach einem der vorhergehenden Ansprüche, **dadurch gekennzeichnet, dass** die erste Schicht (11) Silikon enthält.

7. Verfahren nach einem der vorhergehenden Ansprüche, **dadurch gekennzeichnet, dass** die mindestens eine zweite Schicht (12) mindestens ein Polymer aus der Gruppe bestehend aus Polyurethan und Silikon-Polyurethan-Copolymer enthält.

8. Verfahren zur Herstellung einer Elektrode für medizinische Anwendungen, vorzugsweise zur Implantation als Bestandteil eines funktionalen Elektrostimulationsgeräts, wobei der Isolationsschlauch (1) nach einem Verfahren nach einem der vorhergehenden Ansprüche hergestellt wird und anschließend der Isolationsschlauch (1) so auf ein Leiterelement aufgebracht wird, dass es diesen außen umgibt und elektrisch isoliert.

## Claims

1. A method for producing an insulation tube (1) for an electrode for medical applications, preferably for implantation as part of a functional electrostimulation device, wherein the insulation tube (1) has a first, substantially hollow-cylindrical layer (11) and at least one second layer (12), which is arranged on the circumferential surface (15) of the first layer (11) and covers this at least in part, **characterised in that** the circumferential surface (15) of the first layer (11) is cleaned, prior to the application of the at least one second layer (12), in a cleaning step by means of a first, liquid cleaning agent at a temperature below 30°C, preferably below 20°C.

2. The method according to Claim 1, **characterised in that** the first cleaning agent is liquid CO₂.

3. The method according to one of the preceding claims, **characterised in that,** following the cleaning step and prior to the application of the at least one second layer (12), an adhesion promoter, which preferably contains at least one compound from the group of silanes, siloxanes and carbinol, is applied to at least part of the circumferential surface (15) of the first layer (11).

4. The method according to one of the preceding claims, **characterised in that,** following the cleaning step and prior to the application of the at least one second layer (12), a plasma pre-treatment of the circumferential surface (15) of the first layer (12) is performed.

5. The method according to one of the preceding claims, **characterised in that** the at least one second layer (12) is applied to the cleaned first layer by means of dipping, spraying or co-extrusion.

6. The method according to one of the preceding claims, **characterised in that** the first layer (11) contains silicone.

7. The method according to one of the preceding claims, **characterised in that** the at least one second layer (12) contains at least one polymer from the group consisting of polyurethane and silicone-polyurethane copolymer.

8. A method for producing an electrode for medical applications, preferably for implantation as part of a functional electrostimulation device, wherein the insulation tube (1) is produced by a method according to one of the preceding claims, and the insulation tube (1) is then applied to a conductor element, such that the insulation tube surrounds the conductor element externally and electrically insulates the conductor element.

## Revendications

1. Procédé de fabrication d'un tuyau d'isolation (1) pour une électrode destinée à des utilisations dans le domaine médical, de préférence pour l'implantation en tant que pièce composante d'un appareil d'électrostimulation fonctionnel, où le tuyau d'isolation (1) présente une première couche (11) dans l'essentiel de forme cylindrique creuse et au moins une deuxième couche (12), qui est disposée sur la surface d'enveloppe (15) de la première couche (11) et recouvre celle-ci au moins partiellement, **caractérisé en ce que** la surface d'enveloppe (15) de la première couche (11) est nettoyée avant l'application de l'au moins une deuxième couche (12) dans une étape de nettoyage au moyen d'un premier produit de nettoyage liquide à une température inférieure à 30°C, de préférence, inférieure à 20°C.

2. Procédé selon la revendication 1, **caractérisé en ce que** le premier produit de nettoyage est du CO₂ liquide.

3. Procédé selon l'une des revendications précédentes, **caractérisé en ce qu'**après l'étape de nettoyage et avant l'application de l'au moins une deuxième couche (12) un agent promoteur d'adhérence, qui contient de préférence au moins un composé faisant partie du groupe constitué par des silanes, des siloxanes et des carbinols, est appliqué sur au moins une partie de la surface d'enveloppe (15) de la première couche (11).

4. Procédé selon l'une des revendications précédentes, **caractérisé en ce qu'**après l'étape de nettoyage et avant l'application de l'au moins une deuxième couche (12) un prétraitement par plasma de la surface d'enveloppe (15) de la première couche (12) est réalisé.

5. Procédé selon l'une des revendications précédentes, **caractérisé en ce que** l'au moins une deuxième couche (12) est appliquée au moyen d'une immersion, d'une pulvérisation sur la surface ou d'une co-extrusion sur la première couche nettoyée.

6. Procédé selon l'une des revendications précédentes, **caractérisé en ce que** la première couche (11) contient un silicone.

7. Procédé selon l'une des revendications précédentes, **caractérisé en ce que** l'au moins une deuxième couche (12) contient au moins un polymère du groupe constitué par le polyuréthane et un copolymère silicone-polyuréthane.

8. Procédé de fabrication d'une électrode pour des utilisations dans le domaine médical, de préférence pour une implantation en tant que partie composante d'un appareil d'électrostimulation fonctionnel, dans lequel le tuyau d'isolation (1) est fabriqué d'après un procédé selon l'une des revendications précédentes et ensuite le tuyau d'isolation (1) est rapporté sur un élément conducteur de sorte qu'il entoure celui-ci par l'extérieur et l'isole électriquement.
